# EUROPÄISCHE PATENTSCHRIFT

(11) **EP 2 240 546 B1**
(45) Veröffentlichungstag und Bekanntmachung des Hinweises auf die Patenterteilung: **12.08.2015**
(21) Anmeldenummer: 09705788.9
(22) Anmeldetag: 27.01.2009
(51) Int. Cl.: C09D 7/12, C09D 5/23, C09D 5/38, A61L 29/18, A61L 29/10, A61L 31/08, A61L 31/18, A61M 25/01, A61L 29/02, A61L 29/08, A61L 31/02, A61L 31/10, A61B 5/055, A61M 25/00, C08K 3/22, C08K 7/18, C08K 9/02

(54) **Verfahren zur Herstellung von mit Eisenoxidnanopartikel beschichtete Instrumente für die Invasivmedizin**
Process for the production of instruments coated with iron oxide nanoparticles for invasive medicine
Procédé de fabrication d'instruments revêtus de nanoparticules d'oxyde de fer destinés à la médecine invasive

(30) Priorität: 28.01.2008 DE 102008006402
(43) Veröffentlichungstag der Anmeldung: 20.10.2010
(73) Patentinhaber: Nano4imaging GmbH, 52074 Aachen (DE)
(72) Erfinder: RUSU, Viurel, Eggelshoven, 6471 KN (NL); BORM, paul, NL-6231 CV Meerssen (NL)
(74) Vertreter: Mann, Volker
(86) Internationale Anmeldenummer: PCT/DE2009/000093
(87) Internationale Veröffentlichungsnummer: WO 2009/094990

(56) Entgegenhaltungen:
- WO-A2-2004/093643
- DE-A1- 19 614 136
- US-A1- 2004 030 379
- US-A1- 2006 249 705

## Beschreibung

Die Erfindung betrifft mit Ferrofluiden beschichtete Instrumente für die Invasivmedizin; die mit Ferrofluiden beschichteten Instrumente sind sichtbar in der Magnetresonanztomographie (MRT).

Heutzutage werden weltweit mehr als 20.000.000 Magnetresonanztomographien (MRI) für verschiedene klinische Indikationen durchgeführt. Im Hinblick auf die wachsende Wichtigkeit des Gebrauchs von Ionenbestrahlung in der Therapie und dem wachsenden Interesse an minimal-invasiven Therapien überrascht es nicht, dass sich die Magnetresonanztomographie seit ca. 1995 langsam im Bereich der Radiologie etabliert und hinausragt. Während die Magnetresonanztomographie ursprünglich für Diagnosebilder entwickelt wurde, wird sie heute zu einem Gerät zur Führung und Auswertung von minimalinvasiven therapeutischen Eingriffen verwendet. Das relativ neue Gebiet der Anwendung beschäftigt sich mit Bereichen wie intra-operative und endovaskular eingesetzten MRI-Verfahren. Minimal-invasive endovaskulare Verfahren spielen eine zunehmend große Rolle in der Patientenbehandlung. Aus vielen Gründen sind die radiologischen Verfahren attraktive Alternativen zu chirurgischen Eingriffen und entsprechenden Behandlungen. Beispiele vaskularer Behandlungen sind die Ballon-Angioplastie, Stent oder Stent-Transplantat-Platzierung, Wickeln eines Aneurysmus, Luftembolie und die lokale Arzneimittelversorgung.

Ein Problem im MRT-Verfahren ist, dass die menschlichen oder tierischen Gefäße, wie z.B. der erkrankter Blutgefäße, vollständig sichtbar gemacht werden, in das die endovsakularen Instrumente eingeführt werden. Zusätzlich ist es von Nutzen, dass die Lage der Instrumente bei einer akzeptablen Bildfrequenz lokalisiert werden kann.

In der medizinischen Diagnostik oder Therapeutik werden solche Methoden als invasiv bezeichnet, die in den Körper eindringen, also z. B. eine Biopsie oder ein Abstrich. Im Zusammenhang mit einem besonders schonenden Operationsverfahren ist die minimal-invasive Chirurgie zu nennen.

Ein Beispiel für Instrumente für die Invasivmedizin sind Katheter, Stents, Zieh- und Führungsdrähte.

Katheter sind Röhrchen oder Schläuche verschiedener Durchmesser aus Kunststoff, Latex, Silikon oder Glas, mit denen Hohlorgane wie Harnblase, Magen, Darm, Gefäße usw., aber auch Ohr und Herz sondiert, entleert, gefüllt oder gespült werden können. Dies geschieht aus diagnostischen (untersuchungsbedingten) oder therapeutischen (behandlungsbedingten) Gründen.

Katheter können beispielsweise als
- Venenkatheter: zentraler Venenkatheter oder Venenverweilkanüle;
- in der Urologie: Katheter dienen in der Urologie der Harnableitung und als Hilfsmittel in der Diagnostik und Therapie. In der Diagnostik dienen sie der Urinentnahme und dem Einbringen von Medikamenten und Kontrastmittel.
- Ureterkatheter: Ableitung des Urins aus der Niere über den Harnleiter in die Harnblase oder nach außen.
- Nephrostomiekatheter: Ableitung des Urins aus dem Nierenbecken durch die Haut nach außen.
- in der Kardiologie: Herzkatheter
- in der Hämatologie: Portkatheter
- in der Anästhesie: Periduralkatheter
- in der Hals-Nasen-Ohren-Heilkunde: Tubenkatheter
- in der Dialysetherapie: Peritonealkatheter zur Durchführung der Peritonealdialyse
eingesetzt werden.

Im Einsatz können die Instrumente gegebenenfalls durch eine schlauchartige Röhre in den Körper eingeführt werden.

Bei dem Einbringen eines Instruments, wie eines Katheters, in den Körper ist eine Überwachung erforderlich, um die Einbringung zu steuern und die Untersuchung oder die Therapie sichtbar zu machen.

Zur Sichtbarmachung von Instrumenten für die Invasivmedizin im menschlichen Körper sind verschiedene Verfahren bekannt.

Aus J. Magn. Resonance Imaging 23, 123 bis 129 (2006) ist bekannt, Instrumente für die Invasivmedizin mit paramagnetischen Partikeln zu beschichten. Als paramagnetisches Material wird Dysprosiumoxid eingesetzt.

In Phys.Med.Biol 51 (2006) N127 bis N137 werden verschiedene paramagnetische Marker zur Beschichtung von Instrumenten für die Invasivmedizin verglichen. Auf Grund der höheren Suszeptibilität wird Dysprosiumoxid ferro- und ferrimagnetischem Material vorgezogen.

In der WO 2005/110217 A1 wird beschrieben, Instrumente für die Invasivmedizin mit nanomagnetischem Material zu beschichten und mit Hilfe der magnetischen Resonanz (MRT) abzubilden. Als nanomagnetische Materialien werden Filme aus FeAl, FeAlO und FeAlN eingesetzt.

In der WO 2005/120598 A1 wird ein Katheterführungsdraht beschrieben, der mit einem Kontrastmittel versehen ist. Als Kontrastmittel wird Eisenpulver verwendet, das eine Korngröße von unter 10 µm aufweist.

In der WO 2007/000148 A2 werden stäbchenförmige Körper (z. B. Instrumente für minimal-invasive Eingriffe) beschrieben, die aus einem oder mehreren Filamenten und einem nicht-ferromagnetischen Matrixwerkstoff bestehen, wobei der Matrixwerkstoff das oder die Filamente umschließt oder miteinander verklebt und eine Dotierung erhält, die magnetresonanztomografische Artefakte erzeugt. Als Dotierung werden Nanopartikel aus Seltenen Erden genannt.

Mit der DE 10 2006 020 402 B3 werden Führungsdrähte für Mikrokatheter offenbart, die in Flüssigkeit suspendierte Ferrofluide oder Diamant-Nanopartikel umfassen.

In der US 2005/0079132 A1 werden medizinische Vorrichtungen beschrieben, die zur Sichtbarmachung im magnetischen Feld nanomagnetische Materialien enthalten.

In der WO 2003/035161 A1 werden bei der Magnetresonanztomographie sichtbare medizinische Vorrichtungen aus polymerem Material offenbart, die mit ferromagnetischem Material mit einem Durchmesser von etwa 0,01 bis etwa 50 µm beschichtet sind.

Die WO 2003/099371 A1 offenbart einen Führungsdraht für Katheter mit in seiner äußeren Beschichtung eingekapselten radiopaque Marker, zum Beispiel Gold, Platin oder Palladium. Auf den Führungsdraht kann noch eine hydrophile Beschichtung aufgebracht sein.

In der Druckschrift WO 2005/030286 A1 werden medizinische Vorrichtungen beschrieben, beispielsweise Stents, die durch Einlagerung von Markern, zum Beispiel Stahlpartikel, im magnetischen Feld sichtbar gemacht werden.

Die US 2004/087933 A1 offenbart einen Katheter-Führungsdraht, der aus einem festen Kern aus einem einstückig durchgehenden polymeren Material geformt ist, vorzugsweise aus Polyetheretherketon (PEEK), und durch Extrusion hergestellt wurde. Der Führungsdraht ist zum distalen Ende hin verjüngt und kann mit einer hydrophilen Beschichtung versehen sein.

In der DE 199 21 088 A1 werden implantierbare Stents aus metallischem und/oder nicht-metallischem Material beschreiben, die mit nanoskaligen Teilchen versehen sind, die einen paramagnetischen Kern und mindestens eine Schale aufweisen, die an dem Kern absorbiert ist.

In der US 2006/249705 A1 werden anorganische, röhrenförmige Strukturen offenbart, beispielsweise Stents, die nanomagnetische Partikel mit einer Größe von weniger als 100 nm umfassen. Die nanomagnetischen Partikel dienen der Verbesserung der Visualisierung der röhrenförmigen Struktur in der Magnetresonanztomographie.

Die US 2005/107870 A1 offenbart ein medizinisches Instrument, das eine erste Beschichtung aus einem biologisch aktiven Material aufweist, die sich auf zumindest einem Teil der Oberfläche des Instruments befindet, sowie eine zweite Beschichtungsschicht, die ein polymeres Material und ein nanomagnetisches Material umfasst, wobei die zweite Schicht auf der ersten Schicht aufgebracht ist.

In der Druckschrift US 2004/210289 A1 wird eine Zusammensetzung beschrieben, umfassend nanomagnetische Partikel, welche eine Partikelgröße von weniger als 100 nm aufweisen und aus drei verschiedenen Atomen aufgebaut sind.

Die US 2004/030379 A1 offenbart medizinische Instrumente, die mit einer ersten Beschichtung versehen sind, welche eine biologisch aktive Substanz umfasst, und mit einer zweiten, auf die erste Beschichtung aufgebrachte Beschichtung, wobei die zweite Beschichtung ein polymeres Material und magnetische Partikel umfasst. Die magnetischen Partikel sollen durch Anlegen eines magnetischen Feld aus ihrer Beschichtung herausgelöst werden und so eine Freisetzung der in der ersten Beschichtung enthaltenen biologisch aktiven Substanz ermöglichen.

Das Dokument US 2005/215874 A1 offenbart ein medizinisches Instrument, umfassend einen biologisch kompatiblen Grundkörper, der mit einem Marker zur Verstärkung der Sichtbarkeit in der Magnetresonanztomographie versehen ist.

Die Patentschriften US 4,989,608 und US 5,154,179 offenbaren einen Katheter, der ein flexibles, röhrenförmiges Element umfasst, in das ferromagnetische Partikel eingebettet sind.

Die GB 2 182 451 offenbart ein Verfahren zur Erzeugung von NMR-Signalen während einer Magnetresonanztomographie-Aufnahme, bei dem die Erzeugung von NMR-Signalen in einem Körperteil, der nicht abgebildet werden soll, durch das Einbringen von magnetischem Material, welches das Magnetfeld stört, in dessen Nähe verhindert wird.

Die DE 196 14 136 A1 offenbart ein Verfahren zur Herstellung agglomeratfreier, nanokristalliner Eisenoxidteilchen mit einer Teilchengröße von nicht mehr als 100 nm, vorzugsweise von nicht mehr als 40 nm und insbesondere nicht mehr als 30 nm, die einen sehr hydrolysebeständigen Überzug auf Silan-Basis aufweisen.

In der Praxis werden heute Marker aus Dysprosiumoxid zur Beschichtung von Instrumenten für die Invasivmedizin eingesetzt.

Nachteilig bei den bekannten Systemen ist, dass nur mit Hilfe von wenig verfügbaren und teuren Materialien, wie Dysprosiumoxid, ein Einsatz in der invasivmedizin möglich ist; die Darstellung der mit Dysprosiumoxid beschichteten Instrumente im MRT-Feld ist nicht voll befriedigend.

Aufgabe der vorliegenden Erfindung ist, ein Verfahren zur Herstellung medizinischer Instrumente zur Verfügung zu stellen, bei denen Eisenoxidteilchen als Markierungsmaterialien Verwendung finden, die eine gleichmäßige Verteilung auf den zu beschichtenden medizinischen Instrumenten gewährleisten, keine Unverträglichkeiten aufweisen und im MRT eine Darstellung von Gewebebereichen in hoher Qualität und hohem Kontrast ergeben.

Erfindungsgemäß wurde ein Verfahren zur Herstellung von mit Ferrofluiden beschichteten Instrumenten für die Invasivmedizin gefunden, das dadurch gekennzeichnet, dass sphärische Eisenoxidnanopartikel, die einen Kern aus Fe₃O₄ beinhalten, der mindestens 90 Gew.-% der Nanopartikel umfasst, Hüllen aus SiO₂ aufweisen, die etwa 3 bis 5 Gew.-% der Nanopartikel ausmachen und in denen eine Restfeuchte von etwa 5 Gew.-% verbleibt, wobei die Dichte der Eisenoxid-Nanopartikel 4,6 g/cm³ beträgt, in THF suspendiert und diese Suspension in einer polyurethanhaltigen Trägerflüssigkeit zu einem Ferrofluid mit einer Dichte von 0,998 g/cm³ und einem Durchmesser der Eisenoxidnanopartikel von 200 nm dispergiert werden, wobei ein Matrixwerkstoff der Instrumente ganz oder teilweise mit dem so erhaltenen Ferrofluid, das einen Gehalt an Eisenoxid-Nanopartikeln von 10 Gew.% aufweist und Eisenoxidnanopartikel in Höhe von 65 x 10¹⁵ pro 100 ml enthält beschichtet und die Trägerflüssigkeit ausgehärtet wird, wobei die getrocknete Beschichtung Eisenoxidnanopartikel im Bereich von 62+/- 5 Gew.-% enthält. Ferrofluide im Rahmen der vorliegenden Erfindung sind im Wesentlichen Eisenoxide enthaltende Flüssigkeiten. Die Ferrofluide bestehen in der Regel aus kleinen magnetischen Partikeln, die in einer Trägerflüssigkeit suspendiert sind. Als Trägerflüssigkeit wird in der Regel bevorzugt ein Lack verwendet, in dem die Ferrofluide stabile Dispersionen bilden. Auf den Instrumenten liegen die Ferrofluide in fester und ausgehärteter Form vor.

Im Rahmen der vorliegenden Erfindung werden Instrumente für die Invasivmedizin bevorzugt, bei denen das Eisenoxid in den Ferrofluiden als Nanopartikel vorliegt.

Die vorliegende Erfindung ist dadurch gekennzeichnet, dass die Eisenoxidnanopartikel in den Ferrofluiden weitgehend kugelförmig vorliegen.

Die vorliegende Erfindung ist dadurch gekennzeichnet, dass die Eisenoxidnanopartikel silanisiert sind.

Gemäß einer anderen, besonders bevorzugten Ausführungsform ist bevorzugt, dass die Nanopartikel in den Ferrofluiden im Wesentlichen aus α (alpha) Fe₃O₄ bestehen.

Die vorliegende Erfindung ist dadurch gekennzeichnet, dass das Ferrofluid aus einer Trägerflüssigkeit besteht, in der Eisenoxidnanopartikel suspendiert sind.

Im besonderen ist bevorzugt, dass die Eisenoxidpartikel in den Ferrofluiden kolloidal in der Trägerflüssigkeit suspendiert sind.

Die vorliegende Erfindung ist dadurch gekennzeichnet, dass in den Ferrofluiden eine Dispersion von Eisenoxid-nanopartikeln in einer Trägerflüssigkeit suspendiert ist.

Die vorliegende Erfindung ist dadurch gekennzeichnet, dass in den Ferrofluiden eine Dispersion von Eisenoxidpartikel in einem aprotischen polaren Lösungsmittel in einer Trägerflüssigkeit suspendiert ist.

Das aprotische polare Lösungsmittel ist Tetrahydrofuran,

Die vorliegende Erfindung ist dadurch gekennzeichnet, dass in den Ferrofluiden die Trägerflüssigkeit ein Lack ist. Im Rahmen der vorliegenden Erfindung wird als Lacke Polyurethane, benutzt.

Die Lacke können weitere übliche Komponenten, wie Lösungsmittel, die nach dem Auftragen abtrocknen, enthalten.

Ferrofluide sind an sich bekannt (Physik in unserer Zeit, 32, 122 bis 127 (2001)).

Die vorliegende Erfindung ist dadurch gekennzeichnet, dass die Ferrofluide die Instrumente für die Invasivmedizin als Markierung ganz oder teilweise bedecken.

Eine besondere Ausführungsform der vorliegenden Erfindung ist dadurch gekennzeichnet, dass die Instrumente für die Invasivmedizin aus einem schlauch- oder stabförmigen Matrixwerkstoff bestehen, der selbst nicht ferromagnetisch ist, und der mit einem Ferrofluid beschichtet ist. Matrixwerkstoffe für Instrumente in der Invasivmedizin, die unter Magnetresonanztomographie verwendet werden, können alle Werkstoffe sein, die in der Praxis für diese Instrumente verwendet werden. Beispielsweise seien Kunststoffe, Latex und Silikone genannt.

Eine besondere Ausführungsform der vorliegenden Erfindung ist dadurch gekennzeichnet, dass der schlauchförmige Matrixwerkstoff einen Katheter oder einen Stent bildet.

Eine besondere Ausführungsform der vorliegenden Erfindung ist dadurch gekennzeichnet, dass der Matrixwerkstoff einen Zieh- oder Führungsdraht bildet.

Eine besondere Ausführungsform der vorliegenden Erfindung ist dadurch gekennzeichnet, dass die Beschichtung des Matrixwerkstoffs mit dem Ferrofluid eine Dicke im Bereich von etwa 10 bis etwa 100 µm aufweist.

Der Anteil an Eisenoxidnanopartikeln in der getrockneten Beschichtung liegt vorzugsweise bei 65+/-5 Gew.-%, bevorzugt bei mehr als 65 Gew.%.

Gegenstand der vorliegenden Erfindung sind auch Instrumenten für die Invasivmedizin, dadurch gekennzeichnet ist, dass sie nach dem erfinderischen Verfahren hergestellt sind.

Eine besondere Ausführungsform des erfindungsgemäßen Verfahrens ist dadurch gekennzeichnet, dass die Beschichtung des Matrixwerkstoffs mit dem Ferrofluid durch Tauchen, Sprühen oder mit einem Auftragegerät (z. B. Spin Coating) erfolgt. Auftragegeräte können beispielsweise Pinsel oder Spatel sein (Beispiel: das Ink-Jet-Verfahren).

Das Ferrofluid kann auf dem Instrument bzw. dessen Hülle aufgetragen werden.

Eine weitere besondere Ausführungsform des erfindungsgemäßen Verfahrens ist dadurch gekennzeichnet, dass die Entfernung von Lösungsmitteln in der Trägerflüssigkeit durch Verdampfen, gegebenenfalls im Vakuum, erfolgt.

Das erfindungsgemäße Verfahren kann beispielsweise wie folgt ausgeführt werden:
Das medizinische Instrument wird einmal oder mehrmals in das Ferrofluid getaucht und danach bis zum Aushärten getrocknet.

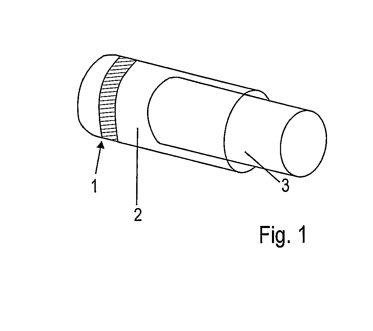

In Figur 1 wird der Aufbau von mit Ferrofluiden beschichteten Instrumenten für die Invasivmedizin beispielsweise wie folgt beschrieben:
Ein Katheter 3 wird in einer schlauchförmigen Hülle 2 geführt. Das Ferrofluid bedeckt die Hülle 2 partiell 1.

Gegenstand der vorliegenden Erfindung ist auch die Verwendung von Instrumenten, die mit Ferrofluiden beschichtet sind, für die Invasivmedizin.

Hierbei wird besonders die Verwendung von Instrumenten, die mit Ferroffuiden beschichtet sind, zur Darstellung im MRT-Feld bei invasivmedizinischen Eingriffen bevorzugt.

Besonders bevorzugt ist die Darstellung von Katheter, Stents, Zieh- oder Führungsdrähte im MRT-Feld.

### Beispiele:

### 1. Herstellung des Ferrofluids

Paramagnetische Eisenoxid-Nanopartikel, die aus α-Fe₃O₄ und/oder Fe₂O₃ mit einer Größe im Bereich von 100 bis 600 nm bestehen und die kugelförmig sind, sind in einem organischen Lösungsmittel wie Tetrahydroforan, Dimethylsulphoxid oder Dioxan dispergiert. Die Dispersion enthält üblicherweise zwischen 10 und 30 Gew.% der paramagnetischen Eisenoxid-Nanopartikel. Diese Dispersion wird mit einem adhesiven Coating-Polymer, zum Beispiel einem Polyurethan, einem Polyolefin, einem Polyacrylat, einem Polystyrol, einem Polyvinyllactam und Co-Polymeren oder Mischungen dieser Polymeren und Co-Polymeren, gemischt.

### 2. Beschichtung des Instruments für die Invasivmedizin

### 2.1. Die Katheter sind mit einem flexiblen Polymer beschichtet, bei dem die Markerpositionen offen sind und der Rest abgedeckt wurde. Die Beschichtung erfolgt durch Tauchen des Katheters in das Ferrofluid nach Beispiel 1.

Die Dicke der Beschichtung auf dem Katheter liegt im Bereich von 10 bis 100 µm und kann durch die Viskosität der polymerhaltigen Dispersion und/oder der Anzahl der Tauchvorgänge kontrolliert werden.

Die Größe der Beschichtung entspricht der Größe der nichtabgedeckten Fläche auf dem Katheter oder der Größe der Feder im Spin-Coating-Verfahren.

Eine weitere Kontrolle erhält man durch die Konzentration der EisenoxidPartikel in den Ferrofluiden.

Typische Tauchzeiten liegen im Bereich von ein bis zwei Minuten und der Katheter soll zwischen den einzelnen Tauchvorgängen ein bis zwei Minuten trocknen.

Das restliche Trocknen erfolgt bei Raumtemperatur während etwa acht Stunden.

Nach dem Trocknen ist das organische Lösungsmittel verdampft und eine ausgehärtete, stabile Beschichtung (Coating) mit dem Ferrofluid verbleibt auf dem Katheter. Zwischen dem flexiblen Polymer und dem Coating besteht eine feste Verbindung.

In das Coating sind die Eisenoxidnanopartikel eingelagert. Um eine Migration von Eisenoxid-Partikeln zu verhindern und/oder die intravaskuläre Einführen des Instruments in das Gefäß zu erleichtern, kann das beschichtete Instrument nochmals mit einem biokompatiblen Polymer überzogen werden (z. B. 0,2 % Chitosan in 1 %iger Essigsäure / 0,1 %iger Polyacrylsäure) oder alternativ einer hydrophilen Beschichtung.

### 2.2. Ziehdrähte werden benötigt, um Katheter oder Implantate an den gewünschten Ort bei einer Operation oder einer Untersuchung zu bringen.

Als Ziehdrähte können Materialien aus Polyvinylchlorid, Polyurethan,

Polyethylenketon, Polyethylen oder Nylon in Kombination mit Fasern oder Nanomaterialien eingesetzt werden. Die Ziehdrähte werden in analoger Weise wie die Katheter beschichtet.

### 3. Darstellung des beschichteten Instruments für die Invasivmedizin im MRI

Durch die Beschichtung mit Ferrofluiden werden Instrumente bei der Invasivmedizin sichtbar gemacht. Die Marker können in verschiedenen Mustern auf den Instrumenten aufgebracht werden, um die Anwendung zu erleichtern. Während der Anwendung ist jederzeit eine Orientierung und Lokalisierung des Instruments möglich. Die Orientierung und Lokalisierung des Instruments erfolgt elektronisch, gegebenenfalls unter Vergrößerung, auf einem Bildschirm. Eine zehnfache Vergrößerung ist beispielsweise ohne Verlust an Bildqualität möglich.

Die Marker erfüllen die folgenden Voraussetzungen im Interventionsbereich:
- Sie sind biokompatibel und sicher.
- Sie sind klein und lassen sich leicht auf die betroffenen Instrumente auftragen, ohne dass deren Anwendbarkeit beeinträchtigt wird.
- Sie lassen sich scharf in MRT darstellen und ermöglichen eine gute Unterscheidung zwischen dem Gewebe und dem Instrument.
- Sie können bei verschiedenen Feldstärken des MRI eingesetzt werden.
- Sie sind passiv und es werden keine Komponenten freigesetzt.

### Ausführungsbeispiele:

### 1. Herstellung der Ferrofluide

Suspension 1: 30 Gew.-% Bayferrox® 318 in Tetrahydrofuran (THF)
Suspension 2: Basecoat (eine Poyurethan enthaltender Lack)

Bei dem von der Firma Lanxess erhältlichen Produkt mit dem Handelsnamen Bayferrox® 318 beziehungsweise Bayferrox® 318 M (die mikroisierte Version von Bayferrox® 318) handelt es sich um sphärische Eisenoxidnanopartikel mit einem Durchmesser von 200 nm, die einen Kern aus Fe₃O₄ aufweisen, der mindestens 90 Gew.-% der Nanopartikel ausmacht, welcher eine Hülle aus SiO₂ aufweist, die etwa 3 bis 5 Gew.-% der Nanopartikel ausmacht. Die verbleibenden etwa 5 Gew.-% fallen auf die in dem kommerziell erhältlichen Produkt enthaltene Restfeuchte. Die Dichte dieser Eisenoxid-Nanopartikel beträgt 4,6 g/cm³.

Es wurden verschiedene Beschichtungsmassen hergestellt, die sich hinsichtlich ihres Gehalts an Eisenoxidnanopartikeln voneinander unterschieden, indem zunächst Suspension 1 und Suspension 2 in einem Verhältnis von 1:2, bezogen auf Gew.-%, miteinander vermischt wurden, um die Beschichtungsmasse M-12 zu erhalten. Die Beschichtungsmasse M-12 wurde anschließend im Verhältnis 1:1, bezogen auf Gew.-%, mit Suspension 2 vermischt, um Beschichtungsmasse M-11 zu erhalten. Zudem wurde Beschichtungsmasse M-11 im Verhältnis 1:1, bezogen auf Gew.-%, mit Suspension 2 vermischt, um Beschichtungsmasse M-10 zu erhalten. Einige Eigenschaften der Beschichtungsmassen sind in Tabelle 1 aufgeführt. Der Anteil an Eisenoxid-Nanopartikeln in der getrockneten Beschichtung wurde nach erfolgter Beschichtung der Führungsdrähte bestimmt.

**Tabelle 1: Eigenschaften der Beschichtungsmassen.**

| Beschichtungsmasse | Dichte (g/cm³) | Gehalt an Nanopartikel (g/100 ml) | Gehalt an Nanopartikel (Anzahl / 100 ml) | Anteil an Eisenoxid-Nanopartikeln in getrockneter Beschichtung (Gew.-%) |
|---|---|---|---|---|
| Suspension 2 | 0,876 | - | - | - |
| M-10 | 0,914 | 2,5 | 16,25 x 10¹⁵ | 12,8 ± 5 |
| M-11 | 0,946 | 5,0 | 132,5 x 10¹⁵ | 31,3 ± 5 |
| M-12 | 0,998 | 10,0 | 65 x 10¹⁵ | 62,0 ± 5 |

### Suspension 2, M-10, M-11 sind nicht erfindungsgemäß.

### 2. Beschichten von Katheter-Führungsdrähten

Drähte aus Polyvinylchlorid (PVC) mit einem Durchmesser von 2 mm und einer Länge von 1 Meter wurden von der Firma Profilplast (Sittard, NL) bezogen. Katheter-Führungsdrähte mit einer Seele aus Polyetheretherketon (PEEK) und einer Ummantelung aus Polyurethan stammten von der Firma Biotronik AG Bülach, CH).

Vor ihrer Beschichtung wurden die Drähte mit 70 Vol.-% Isopropanol (in Wasser) gesäubert. Mit der Spitze einer Graphitmiene, die unmittelbar zuvor in die jeweilige Beschichtungsmasse getunkt worden war, wurde der zu beschichtende Draht an einer Stelle berührt und anschließend gedreht, so dass eine ringförmige, quer zur Längsachse des Drahtes verlaufende, sichtbare Bande auf dem Führungsdraht erhalten wurde. Auf diese Weise wurden pro Draht mehrere Banden aufgebracht, die einen vorbestimmten Abstand zueinander aufwiesen.

Die beschichteten Drähte wurden für 24 Stunden bei Raumtemperatur getrocknet, bevor ihre Oberfläche durch vorsichtiges Abreiben mit destilliertem Wasser gereinigt wurde.

### 3. Sichtbarkeit der beschichteten Führungsdrähte im MRT

Die beschichteten PVC-Drähte wurden in einer MnCl₂-Lösung (*T1*/*T2* = *1030*/*140* ms bei *1,5 T*) mit Hilfe eines 1,5 Tesla Magnetresonanz-Tomographen und Verwendung des "FE tracking sequence PassTrack" untersucht.

Bei dieser Untersuchung waren alle beschichteten Drähte im MRI sichtbar. Die mit M-10 beschichteten Drähte führten zu den besten Aufnahmen, das heißt zu den deutlichsten Abbildungen der Markierungen. Die mit M-12 beschichteten Drähte führten zu sehr dunklen Abbildungen. Offensichtlich war die Konzentration der Eisenoxid-Nanopartikel zu hoch, um bei diesem Versuchsansatz zu deutlichen Abbildungen zu führen.

Die Nützlichkeit von beschichteten PEEK-Führungsdrähten wurde unter Verwendung eines 1,5 T Ganzkörper-Tomographen (Espreee^{®}, Siemens Medical Solutions, Erlangen, DE), der mit einem Hochleistungsgradientensystem (Gradientenstärke: 33 mT/m; Schwenkrate: 100 T/m/s) ausgestattet war, untersucht. Hierbei wurden die Nierenarterien sowie die Vena cava von zwei Scheinen sondiert. Das Sondieren mit den Führungsdrähten wurde unter Realzeit beobachtet. Die Sichtbarkeit der Führungsdrähte, ihre Beweglichkeit und ihre Führbarkeit wurde von dem durchführenden Radiologen bewertet. Die Sichtbarkeit der mit Beschichtungsmasse M-12 beschichteten Führungsdrähte war ausgezeichnet. Die Beweglichkeit und Führbarkeit der Führungsdrähte war gut und einem kommerziell erhältlichen Standard (Terumo Glidewire Stiff and Standard) zumindest ebenbürtig.

### 4. Vergleich unterschiedlicher Markierungsmaterialien

Um die Qualität der Sichtbarkeit von mit M-12 beschichteten PEEK-Führungsdrähten beurteilen zu können, wurden Vergleichsversuche durchgeführt, bei denen PEEK-Führungsdrähte mit unterschiedlichen magnetischen Materialien beschichtet und in einem in vitro-Versuch an Aorta-Phatomen untersucht wurden.

Zu diesem Zweck wurden die in Tabelle 2 angegebenen magnetischen Materialien in THF in der gleichen Konzentration wie für die Herstellung der Beschichtungsmasse M-12 (Beispiel 1) suspendiert. Die Beschichtung der Führungsdrähte erfolgte wie in Beispiel 2 beschrieben.

**Tabelle 2: Übersicht über die im Vergleichsversuch verwendeten metallischen Nanopartikel**

| | ***Magnetisches Material*** | ***Bezugsquelle*** |
|---|---|---|
| 1 | Mischung aus Maghemit und Magnetit Fe₂O₃;Größenbereich: 150-300 nm | Dry MagFerro (MagnaMedics ferrofluid), Lot: MF08010801 |
| 2 | Gadolinium-III-Oxid, Nanopartikel, 10 - 100 nm, 99,9 % | Aldrich, 637335-10G |
| 3 | Fe₃O₄ | Bayferrox 318 M, Lanxess |
| 4 | Fe (II, III) oxid Nanopartikel, 10-100 nm, | 99,95% Alfa Aesar 044120 |
| 5 | Fe III oxid, gamma, Nanopartikel, 10-100 nm, 99,95 % | Alfa Aesar 039951 |
| 6 | Fe₂O₃ 200 nm, 96,0 % | Bayoxide E 8707H, Lanxess |

Bei den Vergleichsversuchen zeigte sich, dass Probe 3, bei der es sich um mit Beschichtungsmasse M-12 beschichtete PEEK-Führungsdrähte handelte, den besten Kontrast und die deutlichste Abbildung der Marker in der Magnetresonanz-Tomographie zeigte, besser als die anderen Eisenoxidnanopartikel aufweisenden Beschichtungen. Alle Eisenoxidnanopartikel aufweisenden Beschichtungen führten zu besseren Abbildungen im MRT als die Markierung mit dem als Standard verwendeten Gadolinium-III-oxid.

## Patentansprüche

1. Verfahren zur Herstellung von mit Ferrofluiden beschichteten Instrumenten für die Invasivmedizin, **dadurch gekennzeichnet, dass** sphärische Eisenoxidnanopartikel , die einen Kern aus Fe₃O₄ beinhalten, der mindestens 90 Gew.-% der Nanopartikel umfasst, Hüllen aus SiO₂ aufweisen, die etwa 3 bis 5 Gew.-% der Nanopartikel ausmachen und in denen eine Restfeuchte von etwa 5 Gew.-% verbleibt, wobei die Dichte der Eisenoxid-Nanopartikel 4,6 g/cm³ beträgt,in THF suspendiert und diese Suspension in einer polyurethanhaltigen Trägerflüssigkeit zu einem Ferrofluid mit einer Dichte von 0,998 g/cm³ und einem Durchmesser der Eisenoxidnanopartikel von 200 nm dispergiert werden, wobei ein Matrixwerkstoff der Instrumente ganz oder teilweise mit dem so erhaltenen Ferrofluid, das einen Gehalt an Eisenoxid-Nanopartikeln von 10 Gew.% aufweist und Eisenoxidnanopartikel in Höhe von 65 x 10¹⁵ pro 100 ml enthält beschichtet und die Trägerflüssigkeit ausgehärtet wird, wobei die getrocknete Beschichtung Eisenoxidnanopartikel im Bereich von 62+/- 5 Gew.-% enthält.

2. Verfahren nach Anspruch 1 , **dadurch gekennzeichnet, dass** der Matrixwerkstoff schlauchförmig ausgebildet ist und einen Katheter, einen Stent und der stabförmige Matrixwirkstoff einen Zieh- oder Führungsdraht oder andere Instrumenten für minimal-invasive Eingriffe bildet.

3. Verfahren nach Anspruch 1 oder 2 , **dadurch gekennzeichnet, dass** der Matrixwerkstoff aus einem Polymer, Metall oder Glas besteht.

4. Verfahren nach einem der vorhergehenden Ansprüche, **dadurch gekennzeichnet, dass** die Beschichtung des Matrixwerkstoffs eine Dicke im Bereich von 10 µm bis 100 µm aufweist.

5. Instrumente für die Invasivmedizin, **dadurch gekennzeichnet, dass** sie nach einem Verfahren gemäß einem der Ansprüche 1 bis 4 hergestellt wurden.

6. Verwendung von Instrumenten nach Anspruch 5 für die Invasivmedizin.

7. Verwendung nach Anspruch 6 von Instrumenten zur Darstellung im MRT-Feld bei invasivmedizinischen Eingriffen.

## Claims

1. A method for producing instruments used in invasive medicine and coated with ferrofluids, **characterized in that** spherical iron oxide nanoparticles having a core of Fe₃O₄ which makes up at least 90 % by weight of the nanoparticles, have a shell of SiO₂, which makes up about 3 to 5 % by weight of the nanoparticles and in which a residual moisture of approximaltely 5 % by weight is remaining, wherein the density of these iron oxide nanoparticles is 4.6 g/cm³, being suspended in tetrahydrofuran and this suspension being dispersed to form a ferrofluid with a density of 0.998 g/cm³ and a diameter of 200 nm in a carrier liquid containing polyurethane, wherein the matrix material of the instruments is coated wholly or partially with the ferrofluid thus obtained, which contains iron oxide nanoparticles with a range of of 10 % by weight and an amount of 65 x 10¹⁵ iron oxide nanoparticles per 100 ml and the carrier liquid is hardened, wherein the dried coating comprises iron oxide nanoparticles in a range of 62 +/- 5 % by weight.

2. The method as claimed in claim 1, **characterized in that** the matrix material is formed rod-shaped like and the rod-shaped matrix material forms a catheter, a stent, a pull wire or guide wire or other instruments for minimally invasive interventions.

3. The method as claimed in claim 1 or 2, **characterized in that** the matrix material is composed of a polymer, metal or glass.

4. The method as claimed in one of the preceding claims, **characterized in that** the coating of the matrix material performs a thickness in the range of 10 µm to 100 µm.

5. Instruments for invasive medicine, **characterized in that** they have been produced by a method as claimed in one of claims 1 to 4.

6. The use of instruments as claimed in claim 5 for invasive medicine.

7. The use of instruments as claimed in claim 6 for visualizing invasive medical interventions in MRT applications.

## Revendications

1. Procédé de production d'instruments, recouverts de ferrofluides, qui sont destinés à la médecine invasive, **caractérisé en ce que** des nanoparticules d'oxyde de fer sphériques, qui contiennent un noyau de Fe₃O₄ comportant au moins 90% en poids de nanoparticules, sont pourvues d'enveloppes de SiO₂ qui sont constituées de 3 à 5% environ de nanoparticules et dans lesquelles la teneur en humidité résiduelle est de 5% en poids environ, la densité des nanoparticules d'oxyde de fer étant de 4,6 g/cm³, sont mises en suspension dans du THF et cette suspension est disposée dans un liquide porteur contenant un polyuréthane pour obtenir un ferrofluide d'une densité de 0,998 g/cm³ et d'un diamètre de nanoparticule d'oxyde de fer de 200 nm, une matière matricielle des instruments étant recouverte en totalité ou en partie du ferrofluide ainsi obtenu, qui a une teneur en nanoparticules d'oxyde de fer de 10% en poids et qui contient des nanoparticules d'oxyde de fer à hauteur de 65 x 10¹⁵ pour 100 ml et le liquide porteur est durci, le revêtement séché contenant des nanoparticules d'oxyde de fer dans la gamme de 62+/-5% en poids.

2. Procédé selon la revendication 1, **caractérisé en ce que** la matière matricielle a la forme d'un tube et forme un cathéter, un stent et la matière matricielle en forme de barre forme un fil de traction ou de guidage ou autres instruments destinés à des interventions invasives minimales.

3. Procédé selon la revendication 1 ou 2, **caractérisé en ce que** la matière matricielle est constituée d'un polymère, d'un métal ou d'un verre.

4. Procédé selon l'une des revendications précédentes, **caractérisé en ce que** le revêtement de la matière matricielle a une épaisseur dans la gamme de 10 µm à 100 µm.

5. Instruments pour la médecine invasive, **caractérisés en ce qu'**ils ont été produits par un procédé selon l'une des revendications 1 à 4.

6. Utilisation d'instruments selon la revendication 5 pour la médecine invasive.

7. Utilisation selon la revendication 6 d'instruments pour la l'exposition dans le champ d'un tomographe à résonance magnétique lors d'interventions en médecine invasive.
